# EUROPEAN PATENT APPLICATION

(11) **EP 4 674 955 A1**
(43) Date of publication of application: **07.01.2026**
(21) Application number: 23924816.4
(22) Date of filing: 29.05.2023
(51) Int. Cl.: C12N 9/16, C12N 15/55, C12P 7/44, C12R 1/21

(54) **ESTERASE MUTANT HAVING POLYESTER DEGRADATION ACTIVITY AND USE THEREOF**

(30) Priority: 27.02.2023 CN 202310168094
(71) Applicant: Tianjin University, Tianjin 300072 (CN)
(72) Inventor: WANG, Zefang, Tianjin 300072 (CN); WANG, Shen, Tianjin 300072 (CN); ZHANG, Hanxiao, Tianjin 300072 (CN); XIAO, Yunjie, Tianjin 300072 (CN); YANG, Haitao, Tianjin 300072 (CN)
(74) Representative: Cabinet Beau de Loménie
(86) International application number: PCT/CN2023/096821
(87) International publication number: WO 2024/178855

(57) **Abstract**

Disclosed in the present invention are esterase mutants having polyester degradation activity and the use thereof. The esterase mutants are one of the following: an esterase A as shown in SEQ ID NO. 3, of which the glutamic acid at the 177th site is mutated into glutamine; or the asparagine at the 178th site is mutated into alanine; or the serine at the 180th site is mutated into threonine, leucine, and valine; or the isoleucine at the 181^{st} site is mutated into valine, etc. Experiments show that a protein expressed by the gene of the esterase mutant of the present invention can be correctly folded and can be purified in a large quantity in an escherichia coli system. In addition, the esterase mutant of the present invention has a function of degrading polyesters, and the thermal stability and the catalytic efficiency of the protein expressed by the gene of the esterase mutant are remarkably improved than those of a wild-type esterase. The esterase mutant of the present invention achieves polyester degrading activity in all the pH range of 4-11. The esterase mutant of the present invention has activity and is stable at 37-70°C.

## Description

### TECHNICAL FIELD

The present invention belongs to the field of genetic engineering of proteins, and particularly relates to an esterase mutant having polyester degradation activity and use thereof.

### BACKGROUND ART

Polyester plastics play a significant role in modern society and are widely used in packaging, construction, textiles, transportation, electronic equipment, industrial machinery, etc., greatly changing human lifestyles. The polyester plastics have excellent properties such as light weight, good insulating properties, high strength and transparency, high thermal performance, and resistance to chemical corrosion. With the extensive use and consumption of polyester plastic products, more and more plastic waste products have been accumulated in environments, which have caused serious damage to global ecological environments and posed a serious threat to human health.

Biological processes have emerged as a novel technology in recent years for the degradation and recycling of polyester plastics. It decomposes such organic matter under the action of biological entities (such as microorganisms, namely bacteria, fungi, and marine microalgae) or enzymes. Biological processes have the advantages of mild process conditions, relatively low energy input, and no need for hazardous chemical reagents and expensive machinery, making them a very promising option. Recently, scientists have reported a novel esterase that can hydrolyze polyesters. Compared with other polyester hydrolyzing enzymes, this esterase can exert its degradation function at a room temperature (equivalent to 30°C). This esterase can degrade commercial high-crystallinity plastic bottles. However, due to obvious deficiencies in catalytic efficiency and thermal stability, it is currently impossible for this esterase to be industrially applied.

### SUMMARY OF THE INVENTION

A first objective of the present invention is to provide esterase mutants having polyester degradation activity to overcome the defects in the prior art.

A second objective of the present invention is to provide recombinant plasmids of encoding genes for esterase mutants having polyester degradation activity.

A third objective of the present invention is to provide recombinant strains containing the above recombinant plasmid.

A fourth objective of the present invention is to provide use of the esterase mutants having polyester degradation activity in polyester hydrolysis.

The technical solutions of the present invention are as follows:
Esterase mutants having polyester degradation activity are provided, where the esterase mutant is one of the following:
MT-1: glutamic acid at the 177^{th} site of the amino acid sequence of an esterase A as shown in SEQ ID NO. 3 is mutated into glutamine;
MT-2: asparagine at the 178^{th} site of the amino acid sequence of an esterase A as shown in SEQ ID NO. 3 is mutated into alanine;
MT-3: serine at the 180^{th} site of the amino acid sequence of an esterase A as shown in SEQ ID NO. 3 is mutated into threonine;
MT-4: serine at the 180^{th} site of the amino acid sequence of an esterase A as shown in SEQ ID NO. 3 is mutated into leucine;
MT-5: serine at the 180^{th} site of the amino acid sequence of an esterase A as shown in SEQ ID NO. 3 is mutated into valine;
MT-6: isoleucine at the 181^{st} site of the amino acid sequence of an esterase A as shown in SEQ ID NO. 3 is mutated into valine;
MT-7: glycine at the 208^{th} site of the amino acid sequence of an esterase A as shown in SEQ ID NO. 3 is mutated into alanine;
MT-8: serine at the 209^{th} site of the amino acid sequence of an esterase A as shown in SEQ ID NO. 3 is mutated into threonine;
MT-9: serine at the 211^{th} site of the amino acid sequence of an esterase A as shown in SEQ ID NO. 3 is mutated into tyrosine;
MT-10: glutamic acid at the 177^{th} site of the amino acid sequence of an esterase A as shown in SEQ ID NO. 3 is mutated into glutamine and serine at the 180^{th} site is mutated into leucine;
MT-11: glutamic acid at the 177^{th} site of the amino acid sequence of an esterase A as shown in SEQ ID NO. 3 is mutated into glutamine and isoleucine at the 181^{st} site is mutated into valine;
MT-12: glutamic acid at the 177^{th} site of the amino acid sequence of an esterase A as shown in SEQ ID NO. 3 is mutated into glutamine and glycine at the 208^{th} site is mutated into alanine;
MT-13: asparagine at the 178^{th} site of the amino acid sequence of an esterase A as shown in SEQ ID NO. 3 is mutated into alanine and glutamic acid at the 177th site is mutated into glutamine;
MT-14: asparagine at the 178^{th} site of the amino acid sequence of an esterase A as shown in SEQ ID NO. 3 is mutated into alanine and serine at the 180^{th} site is mutated into leucine;
MT-15: asparagine at the 178^{th} site of the amino acid sequence of an esterase A as shown in SEQ ID NO. 3 is mutated into alanine and isoleucine at the 181^{st} site is mutated into valine;
MT-16: asparagine at the 178^{th} site of the amino acid sequence of an esterase A as shown in SEQ ID NO. 3 is mutated into alanine and glycine at the 208^{th} site is mutated into alanine;
MT-17: asparagine at the 178^{th} site of the amino acid sequence of an esterase A as shown in SEQ ID NO. 3 is mutated into alanine and serine at the 209^{th} site is mutated into threonine;
MT-18: serine at the 180^{th} site of the amino acid sequence of an esterase A as shown in SEQ ID NO. 3 is mutated into threonine and glutamic acid at the 177^{th} site is mutated into glutamine;
MT-19: serine at the 180^{th} site of the amino acid sequence of an esterase A as shown in SEQ ID NO. 3 is mutated into threonine and asparagine at the 178^{th} site is mutated into alanine;
MT-20: serine at the 180^{th} site of the amino acid sequence of an esterase A as shown in SEQ ID NO. 3 is mutated into threonine and isoleucine at the 181^{st} site is mutated into valine;
MT-21: serine at the 180^{th} site of the amino acid sequence of an esterase A as shown in SEQ ID NO. 3 is mutated into threonine and glycine at the 208^{th} site is mutated into alanine;
MT-22: serine at the 180^{th} site of the amino acid sequence of an esterase A as shown in SEQ ID NO. 3 is mutated into threonine and serine at the 209^{th} site is mutated into threonine;
MT-23: serine at the 180^{th} site of the amino acid sequence of an esterase A as shown in SEQ ID NO. 3 is mutated into threonine and serine at the 211^{th} site is mutated into tyrosine;
MT-24: serine at the 180^{th} site of the amino acid sequence of an esterase A as shown in SEQ ID NO. 3 is mutated into leucine and isoleucine at the 181^{st} site is mutated into valine;
MT-25: serine at the 180^{th} site of the amino acid sequence of an esterase A as shown in SEQ ID NO. 3 is mutated into leucine and glycine at the 208^{th} site is mutated into alanine;
MT-26: serine at the 180^{th} site of the amino acid sequence of an esterase A as shown in SEQ ID NO. 3 is mutated into valine and glutamic acid at the 177^{th} site is mutated into glutamine;
MT-27: serine at the 180^{th} site of the amino acid sequence of an esterase A as shown in SEQ ID NO. 3 is mutated into valine and asparagine at the 178^{th} site is mutated into alanine;
MT-28: serine at the 180^{th} site of the amino acid sequence of an esterase A as shown in SEQ ID NO. 3 is mutated into valine and isoleucine at the 181^{st} site is mutated into valine;
MT-29: serine at the 180^{th} site of the amino acid sequence of an esterase A as shown in SEQ ID NO. 3 is mutated into valine and glycine at the 208^{th} site is mutated into alanine;
MT-30: serine at the 180^{th} site of the amino acid sequence of an esterase A as shown in SEQ ID NO. 3 is mutated into valine and serine at the 209^{th} site is mutated into threonine;
MT-31: isoleucine at the 181^{st} site of the amino acid sequence of an esterase A as shown in SEQ ID NO. 3 is mutated into valine and glycine at the 208^{th} site is mutated into alanine;
MT-32: serine at the 209^{th} site of the amino acid sequence of an esterase A as shown in SEQ ID NO. 3 is mutated into threonine and glutamic acid at the 177^{th} site is mutated into glutamine;
MT-33: serine at the 209^{th} site of the amino acid sequence of an esterase A as shown in SEQ ID NO. 3 is mutated into threonine and serine at the 180^{th} site is mutated into leucine;
MT-34: serine at the 209^{th} site of the amino acid sequence of an esterase A as shown in SEQ ID NO. 3 is mutated into threonine and isoleucine at the 181^{st} site is mutated into valine;
MT-35: serine at the 209^{th} site of the amino acid sequence of an esterase A as shown in SEQ ID NO. 3 is mutated into threonine and glycine at the 208^{th} site is mutated into alanine;
MT-36: serine at the 211^{th} site of the amino acid sequence of an esterase A as shown in SEQ ID NO. 3 is mutated into tyrosine and glutamic acid at the 177^{th} site is mutated into glutamine;
MT-37: serine at the 211^{th} site of the amino acid sequence of an esterase A as shown in SEQ ID NO. 3 is mutated into tyrosine and asparagine at the 178^{th} site is mutated into alanine;
MT-38: serine at the 211^{th} site of the amino acid sequence of an esterase A as shown in SEQ ID NO. 3 is mutated into tyrosine and serine at the 180^{th} site is mutated into leucine;
MT-39: serine at the 211^{th} site of the amino acid sequence of an esterase A as shown in SEQ ID NO. 3 is mutated into tyrosine and serine at the 180^{th} site is mutated into valine;
MT-40: serine at the 211^{th} site of the amino acid sequence of an esterase A as shown in SEQ ID NO. 3 is mutated into tyrosine and isoleucine at the 181^{st} site is mutated into valine;
MT-41: serine at the 211^{th} site of the amino acid sequence of an esterase A as shown in SEQ ID NO. 3 is mutated into tyrosine and glycine at the 208^{th} site is mutated into alanine; and
MT-42: serine at the 211^{th} site of the amino acid sequence of an esterase A as shown in SEQ ID NO. 3 is mutated into tyrosine and serine at the 209^{th} site is mutated into threonine.

Compared with the amino acid sequence of the esterase A as shown in SEQ ID NO. 3, involves the mutation sites involved in the amino acid sequence of the esterase mutant include at least three of the amino acid mutation sites.

Recombinant plasmids of encoding genes for the above mentioned esterase mutants having polyester degradation activity are provided.

Recombinant strains containing the above recombinant plasmids are provided.

Use of the above esterase mutants having polyester degradation activity in polyester hydrolysis are provided.

The present invention has the beneficial effects:
It is shown experimentally that the proteins expressed by the genes of the present invention's esterase mutants can be correctly folded and can be purified in large quantity of in an Escherichia coli (E. coli) system. Meanwhile, the esterase mutants of the present invention have the function of degrading polyesters, and the thermal stability and catalytic efficiency of the proteins expressed by the esterase mutant gene are significantly improved compared with those of a wild-type esterase (SEQ ID NO. 1). The esterase mutants of the present invention achieves polyester degradation activity in all the pH range of 4 to 11. The esterase mutants of the present invention have activity and stability at 37°C to 70°C. Specifically, the capacity for degrading polyesters is significantly improved at a pH of 9.0 and temperature of 50°C compared to that of the wild-type esterase.

### DETAILED DESCRIPTION OF THE EMBODIMENTS

A wild-type esterase is derived from *Ideonella sakaiensis 201-F6,* with the amino acid sequence accession number of UniProtKB: A0A0K8P6T7.1, SEQ ID NO. 2 (WT) is obtained after codon optimization by E. coli, and the amino acid sequence of the wild-type esterase is SEQ ID NO. 1, which is artificially synthesized.

An esterase A is formed by mutating an amino acid at the 94^{th} site of the amino acid sequence as shown in SEQ ID NO. 1 from serine to glutamic acid, mutating an amino acid at the 159^{th} site from aspartic acid to histidine, mutating an amino acid at the 206^{th} site from asparagine to cysteine, mutating an amino acid at the 215^{th} site from serine to threonine, mutating an amino acid at the 219^{th} site from asparagine to aspartic acid, and mutating an amino acid at the 255^{th} site from asparagine to cysteine, the amino acid sequence thereof is as shown in SEQ ID NO. 3, and a nucleotide sequence of the esterase A is as shown in SEQ ID NO. 4.

An expression vector pET-21b (+) is commercially available, and E. coli DH5 alpha and BL21 (DE3) are commercially available.

### Experimental materials:

Luria-Bertani (LB) broth: 10 g of peptone, 5 g of yeast extract, and 10 g of NaCl were diluted with double distilled water to 1 L, and autoclaved at 121°C and 0.1 MPa for 20 minutes.

LB agar: 10 g of peptone, 5 g of yeast extract, 10 g of NaCl, and 15 g of agar powder were diluted with double distilled water to 1 L, and autoclaved at 121°C and 0.1 MPa for 20 minutes.

Isopropyl-beta-D-thiogalactopyranoside (IPTG): 10 g of IPTG powder was dissolved in 42 mL of sterile double distilled water in a sterile environment and mixed thoroughly.

SDS-PAGE (polyacrylamide gel electrophoresis), 12% SDS-PAGE resolving gel thereof is shown in Table 1, and SDS-PAGE stacking gel thereof is shown in Table 2.

10% APS (ammonium persulfate) solution: 1 g of APS solid powder was diluted with sterile water to 10 mL so as to be dissolved thoroughly.

10% SDS (sodium lauryl sulphate) solution: 1 g of SDS solid powder was diluted with sterile water to 10 mL so as to be dissolved thoroughly.

30% acrylamide solution (100 mL): 30 g of acrylamide and 0.8 g of methylene diacrylamide were diluted with double distilled water to 100 mL.

1.5 M Tris-HCl buffer solution (pH=8.8, 1 L): 181.71 g of Tris was weighed and dissolved in 800 mL of purified water, pH was adjusted to 8.8, and the volume was filled up to 1 L.

0.5 M Tris-HCl buffer solution (pH=6.8, 500 mL): 60.57 g of Tris was weighed and dissolved in 400 mL of purified water, pH was adjusted to 6.8, and the volume was filled up to 500 mL.

**Table 1 12% SDS-PAGE resolving gel**

| Component | Volume (mL) |
|---|---|
| 30% acrylamide | 4.0 |
| 1.5 M Tris-HCl pH 8.8 | 2.5 |
| 10% SDS | 0.1 |
| 10% APS | 0.1 |
| TEMED | 0.005 |
| Double distilled water | 3.3 |

**Table 2 SDS-PAGE stacking gel**

| Component | Volume (mL) |
|---|---|
| 30% acrylamide | 0.83 |
| 1.5 M Tris-HCl pH 6.8 | 0.63 |
| 10% SDS | 0.05 |
| 10% APS | 0.05 |
| TEMED | 0.005 |
| Double distilled water | 3.4 |

5×Tris-glycine electrophoresis buffer solution (5 L): 75.5 g of Tris, 470 g of glycine, and 25 g of SDS were dissolved in purified water and diluted to 5 L.

Phosphate buffer solution (pH 2.5): 0.02 M NaH₂PO₄ was prepared, pH was adjusted to 2.5 with H₃PO₄, and finally the volume was filled up to 1 L.

Stop buffer solution: 75 mL of phosphate buffer solution (pH 2.5) and 25 mL of methanol.

The present invention will be further described below with reference to examples, and the following is only part of preferred examples of the present invention, instead of limiting the present invention to other forms. Any amendment or equivalent change made to the examples according to the technical substance of the present invention without departing from the content of the solutions of the present invention falls within the scope of protection of the present invention.

### Example 1

### Construction of recombinant plasmid of esterase mutant MT-1 having polyester degradation activity

A nucleotide sequence SEQ ID NO. 4 of an artificially synthesized esterase A was adopted as a template, MT-1-F was adopted as a forward primer (SEQ ID NO. 5), MT-1-R (SEQ ID NO. 6) was adopted as a reverse primer, and a sequence containing a mutant encoding gene was obtained after polymerase chain reaction (PCR). This product was subsequently ligated and recombined using a seamless cloning enzyme to obtain a recombinant plasmid containing an esterase mutant MT-1 gene.

The specific steps are as follows:
After the nucleotide sequence SEQ ID NO. 4 of the esterase A was obtained after design and gene synthesis, the primers were firstly designed for PCR, the forward primer and the reverse primer were named MT-1-F (SEQ ID NO. 5) and MT-1-R (SEQ ID NO. 6) respectively, a target gene was amplified, and a PCR system is shown in Table 3.

**Table 3 PCR system**

| Component | Volume (µL) |
|---|---|
| Template | 1 |
| Forward primer (10 µM) | 5 |
| Reverse primer (10 µM) | 5 |
| 10×polymerase buffer solution | 10 |
| 10 mM dNTPs (2.5 mM each) | 8 |
| Polymerase | 2 |
| Double distilled water | 69 |

Conditions of a PCR reaction: predegeneration at 95°C for 3 min, degeneration at 95°C for 30 s, annealing at a set temperature gradient ranging from 50°C to 60°C for 30 s, extension at 72°C for 30 s, 33 cycles, and finally extension for another 5 min to complete the reaction. After the reaction was completed, electrophoresis was performed with 1% agarose gel, then the gel was recovered with a gel recovery kit, and a PCR product was obtained.

The PCR product contains an original template plasmid of the esterase A, and it was essential to perform Dpn I digestion prior to recombinant cyclization in order to prevent the formation of a false-positive transformant after transformation. A methylated template plasmid was removed, and a digestion product was obtained. A Dpn I digestion system was shown in Table 4.

As for discontinuous polybasic site-directed mutation, a following ligation system (as shown in Table 5) was prepared on ice: the reaction mixture was gently pipetted up and down by a pipette to be mixed thoroughly (instead of mixing thoroughly with shaking), and briefly centrifuged to collect reaction liquid to the bottom of a tube. The reaction liquid was kept at 37°C for 30 min and then cooled to 4°C or cooled on the ice immediately.

**Table 4 Dpn 1 digestion system**

| Component | Volume (µL) |
|---|---|
| Plasmid or PCR product | 10 (about 1 µg) |
| 10×buffer solution | 3 |
| Dpn I enzyme | 1 |
| Double distilled water | 16 |

**Table 5 Ligation system**

| Component | Volume (µL) |
|---|---|
| n Dpn I digestion products | 1 |
| 5×buffer solution | 4 |
| Seamless cloning enzyme | 2 |
| Double distilled water | 3 |

After a ligation reaction was completed, 20 µL of ligation product was added to 100 µL of E. coli DH5 alpha competent cells, stood still on ice for 30 min, then was subjected to heat shock in a water bath at 42°C for 90 s, was quickly inserted into the ice, and stood still for 5 min, 500 µL of LB broth was added, and the product resuscitated in a shaker at 37°C for 45 min. Resuscitated bacteria were centrifuged at 3,000 rpm for 4 min, part of supernatant was pipetted and removed, about 100 µL of supernatant was reserved, and the bacteria were gently mixed thoroughly with it, evenly coated onto an LB agar containing 100 µg/mL ampicillin with a coating bar, and incubated overnight in an incubator at 37°C. Single colonies were picked into 5 mL of LB broth containing 100 µg/mL ampicillin on the next day, and subjected to shaking culture in the shaker at 37°C for 10-12 h. Plasmids were extracted with a MiniPrep Kit (Tiangen), then the extracted plasmids were subjected to double enzyme digestion verification, a double enzyme digestion verification system is shown in Table 6, the reaction system was put into the water bath at 37°C for reaction for 30 min, agarose gel electrophoresis was performed after the reaction was completed, and plasmids with correct enzyme digestion verification were sent to a sequencing company for sequencing verification.

**Table 6 Double enzyme digestion verification system**

| Component | Volume (µL) |
|---|---|
| Recombinant plasmid | 7 |
| 10×Fast digest enzyme buffer solution | 1 |
| Fast digest enzyme *Ndel* | 1 |
| Fast digest enzyme *Xho*I | 1 |

The recombinant plasmid pET-21b-MT-1 of the esterase mutant MT-1 having polyester degradation activity was obtained through verification.

The recombinant plasmids were prepared according to the method in this example by adopting the nucleotide sequence SEQ ID NO. 4 of the artificially synthesized esterase A as a template and corresponding primer pairs shown in Table 7 as primers:

pET-21b-MT-2, pET-21b-MT-3, pET-21b-MT-4, pET-21b-MT-5, pET-21b-MT-6, pET-21b-MT-7, pET-21b-MT-8, pET-21b-MT-9, pET-21b-MT-10, pET-21b-MT-11, pET-21b-MT-13, pET-21b-MT-14, pET-21b-MT-15, pET-21b-MT-18, pET-21b-MT-19, pET-21b-MT-20, pET-21b-MT-24, pET-21b-MT-26, pET-21b-MT-27, pET-21b-MT-28, pET-21b-MT-35, pET-21b-MT-41, and pET-21b-MT-42.

**Table 7 Primer sequence of esterase mutant**

| Primer name of esterase mutants | Primer sequences |
|---|---|
| MT-1-F (SEQ ID NO. 5) | tgcgtgccagaacgatagcattgcgccggtga |
| MT-1-R (SEQ ID NO. 6) | tatcgttctggcacgcaaaaatcagggtcggc |
| MT-2-F (SEQ ID NO. 11) | tgcgaagccgatagcattgcgccggtgaacag |
| MT-2-R (SEQ ID NO. 12) | atgctatcggcttcgcacgcaaaaatcagggt |
| MT-3-F (SEQ ID NO. 13) | aaacgatacgattgcgccggtgaacagcagcg |
| MT-3-R (SEQ ID NO. 14) | gcgcaattgcatcgttttcgcacgcaaaaatc |
| MT-4-F (SEQ ID NO. 15) | aaacgatctgattgcgccggtgaacagcagcg |
| MT-4-R (SEQ ID NO. 16) | gcgcaatcagatcgttttcgcacgcaaaaatc |
| MT-5-F (SEQ ID NO. 17) | aaacgatgtgattgcgccggtgaacagcagcg |
| MT-5-R (SEQ ID NO. 18) | gcgcaatcacatcgttttcgcacgcaaaaatc |
| MT-6-F (SEQ ID NO. 19) | aaacgattcggttgcgccggtgaacagcagcg |
| MT-6-R (SEQ ID NO. 20) | gcgcaaccgaatcgttttcgcacgcaaaaatc |
| MT-7-F (SEQ ID NO. 21) | aaatttgcggcgcgagccatagctgcgcgaaca |
| MT-7-R (SEQ ID NO. 22) | gctcgcgccgcaaatttccagaaactgtttcg |
| MT-8-F (SEQ ID NO. 23) | aaatttgcggcggcacccatagctgcgcgaacaccg |
| MT-8-R (SEQ ID NO. 24) | ggtgccgccgcaaatttccagaaactgtttcg |
| MT-9-F (SEQ ID NO. 25) | cagccattattgcgcgaacaccggtaatagcg |
| MT-9-R (SEQ ID NO. 26) | tcgcgcaataatggctgccgccgcaaatttcc |
| MT-10-F (SEQ ID NO. 27) | cgtgccaaaacgatctgattgcgccggtgaacagc |
| MT-10-R (SEQ ID NO. 28) | tcagatcgttttggcacgcaaaaatcagggtcg |
| MT-11-F (SEQ ID NO. 29) | ccagaacgatagcgtggcgccggtgaacagcagc |
| MT-11-R (SEQ ID NO. 30) | ccacgctatcgttctggcacgcaaaaatcagggtcg |
| MT-13-F (SEQ ID NO. 31) | tgccaggcggatagcattgcgccggtg |
| MT-13-R (SEQ ID NO. 32) | aatgctatccgcctggcacgcaaaaatcagggtcg |
| MT-14-F (SEQ ID NO. 33) | cgaagcggatctgattgcgccggtgaacagc |
| MT-14-R (SEQ ID NO. 34) | caatcagatccgcttcgcacgcaaaaatcagg |
| MT-15-F (SEQ ID NO. 35) | tgcgaagcggatagcgtggcgccggtgaacagcagc |
| MT-15-R (SEQ ID NO. 36) | cacgctatccgcttcgcacgcaaaaatcagg |
| MT-18-F (SEQ ID NO. 37) | gtgccagaacgataccattgcgccggtgaacagc |
| MT-18-R (SEQ ID NO. 38) | atggtatcgttctggcacgcaaaaatcagggtcg |
| MT-19-F (SEQ ID NO. 39) | cgaagccgataccattgcgccggtgaacagc |
| MT-19-R (SEQ ID NO. 40) | caatggtatcggcttcgcacgcaaaaatcagg |
| MT-20-F (SEQ ID NO. 41) | tgcgaaaacgataccgtggcgccggtgaacagcagc |
| MT-20-R (SEQ ID NO. 42) | cacggtatcgttttcgcacgcaaaa |
| MT-24-F (SEQ ID NO. 43) | tgcgaaaacgatctggtggcgccggtgaacagcagc |
| MT-24-R (SEQ ID NO. 44) | caccagatcgttttcgcacgcaaaa |
| MT-26-F (SEQ ID NO. 45) | gtgccagaacgatgtgattgcgccggtgaacagc |
| MT-26-R (SEQ ID NO. 46) | atcacatcgttctggcacgcaaaaatcagggtcg |
| MT-27-F (SEQ ID NO. 47) | cgaagcggatgtgattgcgccggtgaacagc |
| MT-27-R (SEQ ID NO. 48) | caatcacatccgcttcgcacgcaaaaatcagg |
| MT-28-F (SEQ ID NO. 49) | tgcgaaaacgatgtggtggcgccggtgaacagcagc |
| MT-28-R (SEQ ID NO. 50) | caccacatcgttttcgcacgcaaaa |
| MT-35-F (SEQ ID NO. 51) | aaatttgcggcgcgacccatagctgcgcgaaca |
| MT-35-R (SEQ ID NO. 52) | ggtcgcgccgcaaatttccagaaactgtttcg |
| MT-41-F (SEQ ID NO. 53) | cggcgcgagccattattgcgcgaacaccggta |
| MT-41-R (SEQ ID NO. 54) | aataatggctcgcgccgcaaatttccagaaactg |
| MT-42-F (SEQ ID NO. 55) | cacccattattgcgcgaacaccggtaatagcg |
| MT-42-R (SEQ ID NO. 56) | tcgcgcaataatgggtgccgccgcaaatttccag |

### Example 2

### Establishment of recombinant plasmid of esterase mutant MT-12 having polyester degradation activity

A nucleotide sequence SEQ ID NO. 4 of an artificially synthesized esterase A was adopted as a template, a short fragment of an encoding gene was amplified by PCR with MT-12-F1 as a forward primer (SEQ ID NO. 7) and MT-12-R2 (SEQ ID NO. 10) as a reverse primer, a long fragment of the encoding gene was amplified by PCR with MT-12-F2 as a forward primer (SEQ ID NO. 9) and MT-12-R1 (SEQ ID NO. 8) as a reverse primer. These two fragments-the short fragment of the encoding gene and the long fragment of the encoding gene-were then ligated and recombined using a seamless cloning enzyme, yielding a recombinant plasmid containing the gene encoding esterase mutant MT-12.

The specific steps are as follows:
After the nucleotide sequence SEQ ID NO. 4 of the esterase A was obtained after design and gene synthesis, the primers were firstly designed for PCR, the short fragment of the encoding gene was amplified by PCR with MT-12-F1 (SEQ ID NO. 7) as the forward primer and MT-12-R2 (SEQ ID NO. 10) as the reverse primer, and the long fragment of the encoding gene was amplified by PCR with MT-12-F2 as the forward primer (SEQ ID NO. 9) and MT-12-R1 (SEQ ID NO. 8) as the reverse primer. A PCR system is shown in Table 3:

Conditions of a PCR reaction were the same as those in Example 1.

The PCR product contains an original template plasmid of the esterase A, and it was essential to perform Dpn I digestion prior to recombinant cyclization in order to prevent the formation of a false-positive transformant after transformation. A methylated template plasmid was removed, and a digestion product was obtained. A Dpn I digestion system was shown in Table 4.

As for discontinuous polybasic site-directed mutation, the following ligation system (as shown in Table 8) was prepared on ice: a product was gently pipetted up and down by a pipette to be mixed thoroughly (instead of mixing thoroughly with shaking), and briefly centrifuged to collect reaction liquid to the bottom of a tube. The reaction liquid was kept at 37°C for 30 min and then cooled to 4°C or cooled on the ice immediately.

**Table 8 Ligation system**

| Component | Volume (µL) |
|---|---|
| Short fragment product of Dpn I digestion encoding gene | 1 |
| Long fragment product of Dpn I digestion encoding gene | 4 |
| 5×buffer solution | 4 |
| Seamless cloning enzyme | 2 |
| Double distilled water | 9 |

After a ligation reaction was completed, 20 µL of ligation product was added to 100 µL of E. coli DH5 alpha competent cells, stood still on ice for 30 min, then was subjected to heat shock in a water bath at 42°C for 90 s, was quickly inserted into the ice, and stood still for 5 min, 500 µL of LB broth was added, and the product resuscitated in a shaker at 37°C for 45 min. Resuscitated bacteria were centrifuged at 3,000 rpm for 4 min, part of supernatant was pipetted and removed, about 100 µL of supernatant was reserved, and the bacteria were gently mixed thoroughly with it, evenly coated onto an LB agar containing 100 µg/mL ampicillin with a coating bar, and incubated overnight in an incubator at 37°C. Single colonies were picked into 5 mL of LB broth containing 100 µg/mL ampicillin on the next day, and subjected to shaking culture in the shaker at 37°C for 10-12 h. Plasmids were extracted with a MiniPrep Kit (Tiangen), then the extracted plasmids were subjected to double enzyme digestion verification, a double enzyme digestion verification system is shown in Table 6, the reaction system was put into the water bath at 37°C for reaction for 30 min, agarose gel electrophoresis was performed after the reaction was completed, and plasmids with correct enzyme digestion verification were sent to a sequencing company for sequencing verification.

The recombinant plasmid pET-21b-MT-12 of the esterase mutant MT-12 having polyester degradation activity was obtained through verification.

The recombinant plasmids were prepared according to the method in this example by adopting the nucleotide sequence SEQ ID NO. 4 of the artificially synthesized esterase A as a template and corresponding primer pairs shown in Table 9 as primers:
pET-21b-MT-16, pET-21b-MT-17, pET-21b-MT-21, pET-21b-MT-22, pET-21b-MT-23, pET-21b-MT-25, pET-21b-MT-29, pET-21b-MT-30, pET-21b-MT-31, pET-21b-MT-32, pET-21b-MT-33, pET-21b-MT-34, pET-21b-MT-36, pET-21b-MT-37, pET-21b-MT-38, pET-21b-MT-39, and pET-21b-MT-40.

**Table 9 Primer sequence of esterase mutant**

| Primer name of esterase mutant | Primer sequence |
|---|---|
| MT-12-F1 (SEQ ID NO. 7) | tgcgtgccagaacgatagcattgcgccg |
| MT-12-R1 (SEQ ID NO. 8) | tatcgttctggcacgcaaaaatcagggtcg |
| MT-12-F2 (SEQ ID NO. 9) | aaatttgcggcgcgagccatagctgcgcgaaca |
| MT-12-R2 (SEQ ID NO. 10) | gctcgcgccgcaaatttccagaaactg |
| MT-16-F1 (SEQ ID NO. 57) | tgcgaagcggatagcattgcgccggtg |
| MT-16-R1 (SEQ ID NO. 58) | atgctatccgcttcgcacgcaaaaatcagg |
| MT-16-F2 (SEQ ID NO. 59) | aaatttgcggcgcgagccatagctgcgcgaaca |
| MT-16-R2 (SEQ ID NO. 60) | gctcgcgccgcaaatttccagaaactg |
| MT-17-F1 (SEQ ID NO. 61) | tgcgaagcggatagcattgcgccggtg |
| MT-17-R1 (SEQ ID NO. 62) | atgctatccgcttcgcacgcaaaaatcagg |
| MT-17-F2 (SEQ ID NO. 63) | aaatttgcggcggcacccatagctgcgcgaacaccg |
| MT-17-R2 (SEQ ID NO. 64) | ggtgccgccgcaaatttccag |
| MT-21-F1 (SEQ ID NO. 65) | aaacgataccattgcgccggtgaacagc |
| MT-21-R1 (SEQ ID NO. 66) | gcgcaatggtatcgttttcgcacgcaaaa |
| MT-21-F2 (SEQ ID NO. 67) | aaatttgcggcgcgagccatagctgcgcgaaca |
| MT-21-R2 (SEQ ID NO. 68) | gctcgcgccgcaaatttccagaaactg |
| MT-22-F1 (SEQ ID NO. 69) | aaacgataccattgcgccggtgaacagc |
| MT-22-R1 (SEQ ID NO. 70) | gcgcaatggtatcgttttcgcacgcaaaa |
| MT-22-F2 (SEQ ID NO. 71) | aaatttgcggcggcacccatagctgcgcgaacaccg |
| MT-22-R2 (SEQ ID NO. 72) | ggtgccgccgcaaatttccag |
| MT-23-F1 (SEQ ID NO. 73) | aaacgataccattgcgccggtgaacagc |
| MT-23-R1 (SEQ ID NO. 74) | gcgcaatggtatcgttttcgcacgcaaaa |
| MT-23-F2 (SEQ ID NO. 75) | cagccattattgcgcgaacaccggtaat |
| MT-23-R2 (SEQ ID NO. 76) | tcgcgcaataatggctgccgccgcaaat |
| MT-25-F1 (SEQ ID NO. 77) | aaacgatctgattgcgccggtgaacagc |
| MT-25-R1 (SEQ ID NO. 78) | gcgcaatcagatcgttttcgcacgcaaaa |
| MT-25-F2 (SEQ ID NO. 79) | aaatttgcggcgcgagccatagctgcgcgaaca |
| MT-25-R2 (SEQ ID NO. 80) | gctcgcgccgcaaatttccagaaactg |
| MT-29-F1 (SEQ ID NO. 81) | aaacgatgtgattgcgccggtgaacagc |
| MT-29-R1 (SEQ ID NO. 82) | gcgcaatcacatcgttttcgcacgcaaaa |
| MT-29-F2 (SEQ ID NO. 83) | aaatttgcggcgcgagccatagctgcgcgaaca |
| MT-29-R2 (SEQ ID NO. 84) | gctcgcgccgcaaatttccagaaactg |
| MT-30-F1 (SEQ ID NO. 85) | aaacgatgtgattgcgccggtgaacagc |
| MT-30-R1 (SEQ ID NO. 86) | gcgcaatcacatcgttttcgcacgcaaaa |
| MT-30-F2 (SEQ ID NO. 87) | aaatttgcggcggcacccatagctgcgcgaacaccg |
| MT-30-R2 (SEQ ID NO. 88) | ggtgccgccgcaaatttccagaaactgtttcg |
| MT-31-F1 (SEQ ID NO. 89) | aaaacgatagcgtggcgccggtgaacagcagc |
| MT-31-R1 (SEQ ID NO. 90) | cgccacgctatcgttttcgcacgca |
| MT-31-F2 (SEQ ID NO. 91) | aaatttgcggcgcgagccatagctgcgcgaaca |
| MT-31-R2 (SEQ ID NO. 92) | gctcgcgccgcaaatttccagaaactgtttcg |
| MT-32-F1 (SEQ ID NO. 93) | tgcgtgccagaacgatagcattgcgccg |
| MT-32-R1 (SEQ ID NO. 94) | tatcgttctggcacgcaaaaatcagggtcg |
| MT-32-F2 (SEQ ID NO. 95) | aaatttgcggcggcacccatagctgcgcgaacaccg |
| MT-32-R2 (SEQ ID NO. 96) | ggtgccgccgcaaatttccag |
| MT-33-F1 (SEQ ID NO. 97) | aaacgatctgattgcgccggtgaacagcagcg |
| MT-33-R1 (SEQ ID NO. 98) | gcgcaatcagatcgttttcgcacgcaaaaatc |
| MT-33-F2 (SEQ ID NO. 99) | aaatttgcggcggcacccatagctgcgcgaacaccg |
| MT-33-R2 (SEQ ID NO. 100) | ggtgccgccgcaaatttccag |
| MT-34-F1 (SEQ ID NO. 101) | aaaacgatagcgtggcgccggtgaacagcagc |
| MT-34-R1 (SEQ ID NO. 102) | cgccacgctatcgttttcgcacgcaaaaatca |
| MT-34-F2 (SEQ ID NO. 103) | aaatttgcggcggcacccatagctgcgcgaacaccg |
| MT-34-R2 (SEQ ID NO. 104) | ggtgccgccgcaaatttccag |
| MT-36-F1 (SEQ ID NO. 105) | tgcgtgccagaacgatagcattgcgccggtga |
| MT-36-R1 (SEQ ID NO. 106) | tatcgttctggcacgcaaaaatcagggtcggc |
| MT-36-F2 (SEQ ID NO. 107) | cagccattattgcgcgaacaccggtaat |
| MT-36-R2 (SEQ ID NO. 108) | tcgcgcaataatggctgccgccgcaaat |
| MT-37-F1 (SEQ ID NO. 109) | tgcgaagccgatagcattgcgccggtgaacag |
| MT-37-R1 (SEQ ID NO. 110) | atgctatcggcttcgcacgcaaaaatcagggt |
| MT-37-F2 (SEQ ID NO. 111) | cagccattattgcgcgaacaccggtaat |
| MT-37-R2 (SEQ ID NO. 112) | tcgcgcaataatggctgccgccgcaaat |
| MT-38-F1 (SEQ ID NO. 113) | aaacgatctgattgcgccggtgaacagcagcg |
| MT-38-R1 (SEQ ID NO. 114) | gcgcaatcagatcgttttcgcacgcaaaaatc |
| MT-38-F2 (SEQ ID NO. 115) | cagccattattgcgcgaacaccggtaat |
| MT-38-R2 (SEQ ID NO. 116) | tcgcgcaataatggctgccgccgcaaat |
| MT-39-F1 (SEQ ID NO. 117) | aaacgatgtgattgcgccggtgaacagcagcg |
| MT-39-R1 (SEQ ID NO. 118) | gcgcaatcacatcgttttcgcacgcaaaaatc |
| MT-39-F2 (SEQ ID NO. 119) | cagccattattgcgcgaacaccggtaat |
| MT-39-R2 (SEQ ID NO. 120) | tcgcgcaataatggctgccgccgcaaat |
| MT-40-F1 (SEQ ID NO. 121) | aaaacgatagcgtggcgccggtgaacagcagc |
| MT-40-R1 (SEQ ID NO. 122) | cgccacgctatcgttttcgcacgcaaaaatca |
| MT-40-F2 (SEQ ID NO. 123) | cagccattattgcgcgaacaccggtaat |
| MT-40-R2 (SEQ ID NO. 124) | tcgcgcaataatggctgccgccgcaaat |

### Example 3

### Expression and purification of esterase mutants having polyester degradation activity

A recombinant plasmid pET-21b-MT-1 was transformed into E. coli BL21 (DE3) and incubated overnight, and single colonies were picked into 5 mL of LB broth containing 100 µg/mL ampicillin and subjected to shaking culture in a shaker at 37°C for 6-8 h (recombinant strain containing pET-21b-MT-1 was obtained); then a bacterial suspension was transferred into 1 L of LB broth containing 100 µg/mL ampicillin and subjected to shaking culture in the shaker at 37°C for 6-8 h, the bacterial suspension was cultured until OD600 was about 0.6, a temperature dropped to 16°C, IPTG was added to a final concentration of 400 µM, and induction culture was performed for 16-18 h. Then bacteria were centrifugally collected, and the collected bacteria were resuspended with suspension buffer (20 mM Tris-HCl, 300 mM NaCl, 10% glycerol, pH 7.5), and then disrupted with a high-pressure disruptor. A bacterial disrupted product was centrifuged at a high speed (18,000 rpm, 4°C, 45 min), and a supernatant was collected, added to nickel affinity chromatography filler balanced with the suspension buffer in advance, and incubated at 4°C for 1 h. After incubation, impure proteins non-specifically binding to a Ni column were completely washed by a wash buffer (20 mM Tris-HCl, 300 mM NaCl, 20 mM imidazole, 10% glycerol, pH 7.5) at 4°C. After the impure proteins were completely washed, target proteins binding to the Ni column were eluted with about 30 mL of eluate (20 mM Tris-HCl, 300 mM NaCl, 300 mM imidazole, 10% glycerol, pH 7.5). The eluate was collected and concentrated with a 10 kDa concentration tube.

Then the target proteins were further purified with a gel filtration chromatography column (Superdex 200 Increase 10/300 GL, GE) through an AKTA pure system. The buffer solution used was 20 mM Tris-HCl, 300 mM NaCl, pH 7.5. The eluted fractions corresponding to the peak positions were collected and verified through protein gel electrophoresis, and the target proteins were collected according to molecular weight sizes.

The recombinant plasmids pET-21b-MT-2, pET-21b-MT-3, pET-21b-MT-4, pET-21b-MT-5, pET-21b-MT-6, pET-21b-MT-7, pET-21b-MT-8, pET-21b-MT-9, pET-21b-MT-10, pET-21b-MT-11, pET-21b-MT-12, pET-21b-MT-13, pET-21b-MT-14, pET-21b-MT-15, pET-21b-MT-16, pET-21b-MT-17, pET-21b-MT-18, pET-21b-MT-19, pET-21b-MT-20, pET-21b-MT-21, pET-21b-MT-22, pET-21b-MT-23, pET-21b-MT-24, pET-21b-MT-25, pET-21b-MT-26, pET-21b-MT-27, pET-21b-MT-28, pET-21b-MT-29, pET-21b-MT-30, pET-21b-MT-31, pET-21b-MT-32, pET-21b-MT-33, pET-21b-MT-34, pET-21b-MT-35, pET-21b-MT-36, pET-21b-MT-37, pET-21b-MT-38, pET-21b-MY-39, pET-21b-MT-40, pET-21b-MT-41, and pET-21b-MT-42 were respectively operated according to the above steps in this example, and corresponding mutant proteins were obtained respectively.
MT-1: glutamic acid at the 177^{th} site of the amino acid sequence of an esterase A as shown in SEQ ID NO. 3 is mutated into glutamine;
MT-2: asparagine at the 178^{th} site of the amino acid sequence of an esterase A as shown in SEQ ID NO. 3 is mutated into alanine;
MT-3: serine at the 180^{th} site of the amino acid sequence of an esterase A as shown in SEQ ID NO. 3 is mutated into threonine;
MT-4: serine at the 180^{th} site of the amino acid sequence of an esterase A as shown in SEQ ID NO. 3 is mutated into leucine;
MT-5: serine at the 180^{th} site of the amino acid sequence of an esterase A as shown in SEQ ID NO. 3 is mutated into valine;
MT-6: isoleucine at the 181^{st} site of the amino acid sequence of an esterase A as shown in SEQ ID NO. 3 is mutated into valine;
MT-7: glycine at the 208^{th} site of the amino acid sequence of an esterase A as shown in SEQ ID NO. 3 is mutated into alanine;
MT-8: serine at the 209^{th} site of the amino acid sequence of an esterase A as shown in SEQ ID NO. 3 is mutated into threonine;
MT-9: serine at the 211^{th} site of the amino acid sequence of an esterase A as shown in SEQ ID NO. 3 is mutated into tyrosine;
MT-10: glutamic acid at the 177^{th} site of the amino acid sequence of an esterase A as shown in SEQ ID NO. 3 is mutated into glutamine and serine at the 180^{th} site is mutated into leucine;
MT-11: glutamic acid at the 177^{th} site of the amino acid sequence of an esterase A as shown in SEQ ID NO. 3 is mutated into glutamine and isoleucine at the 181^{st} site is mutated into valine;
MT-12: glutamic acid at the 177^{th} site of the amino acid sequence of an esterase A as shown in SEQ ID NO. 3 is mutated into glutamine and glycine at the 208^{th} site is mutated into alanine;
MT-13: asparagine at the 178^{th} site of the amino acid sequence of an esterase A as shown in SEQ ID NO. 3 is mutated into alanine and glutamic acid at the 177^{th} site is mutated into glutamine;
MT-14: asparagine at the 178^{th} site of the amino acid sequence of an esterase A as shown in SEQ ID NO. 3 is mutated into alanine and serine at the 180^{th} site is mutated into leucine;
MT-15: asparagine at the 178^{th} site of the amino acid sequence of an esterase A as shown in SEQ ID NO. 3 is mutated into alanine and isoleucine at the 181^{st} site is mutated into valine;
MT-16: asparagine at the 178^{th} site of the amino acid sequence of an esterase A as shown in SEQ ID NO. 3 is mutated into alanine and glycine at the 208^{th} site is mutated into alanine;
MT-17: asparagine at the 178^{th} site of the amino acid sequence of an esterase A as shown in SEQ ID NO. 3 is mutated into alanine and serine at the 209^{th} site is mutated into threonine;
MT-18: serine at the 180^{th} site of the amino acid sequence of an esterase A as shown in SEQ ID NO. 3 is mutated into threonine and glutamic acid at the 177^{th} site is mutated into glutamine;
MT-19: serine at the 180^{th} site of the amino acid sequence of an esterase A as shown in SEQ ID NO. 3 is mutated into threonine and asparagine at the 178^{th} site is mutated into alanine;
MT-20: serine at the 180^{th} site of the amino acid sequence of an esterase A as shown in SEQ ID NO. 3 is mutated into threonine and isoleucine at the 181^{st} site is mutated into valine;
MT-21: serine at the 180^{th} site of the amino acid sequence of an esterase A as shown in SEQ ID NO. 3 is mutated into threonine and glycine at the 208^{th} site is mutated into alanine;
MT-22: serine at the 180^{th} site of the amino acid sequence of an esterase A as shown in SEQ ID NO. 3 is mutated into threonine and serine at the 209^{th} site is mutated into threonine;
MT-23: serine at the 180^{th} site of the amino acid sequence of an esterase A as shown in SEQ ID NO. 3 is mutated into threonine and serine at the 211^{th} site is mutated into tyrosine;
MT-24: serine at the 180^{th} site of the amino acid sequence of an esterase A as shown in SEQ ID NO. 3 is mutated into leucine and isoleucine at the 181^{st} site is mutated into valine;
MT-25: serine at the 180^{th} site of the amino acid sequence of an esterase A as shown in SEQ ID NO. 3 is mutated into leucine and glycine at the 208^{th} site is mutated into alanine;
MT-26: serine at the 180^{th} site of the amino acid sequence of an esterase A as shown in SEQ ID NO. 3 is mutated into valine and glutamic acid at the 177^{th} site is mutated into glutamine;
MT-27: serine at the 180^{th} site of the amino acid sequence of an esterase A as shown in SEQ ID NO. 3 is mutated into valine and asparagine at the 178^{th} site is mutated into alanine;
MT-28: serine at the 180^{th} site of the amino acid sequence of an esterase A as shown in SEQ ID NO. 3 is mutated into valine and isoleucine at the 181^{st} site is mutated into valine;
MT-29: serine at the 180^{th} site of the amino acid sequence of an esterase A as shown in SEQ ID NO. 3 is mutated into valine and glycine at the 208^{th} site is mutated into alanine;
MT-30: serine at the 180^{th} site of the amino acid sequence of an esterase A as shown in SEQ ID NO. 3 is mutated into valine and serine at the 209^{th} site is mutated into threonine;
MT-31: isoleucine at the 181^{st} site of the amino acid sequence of an esterase A as shown in SEQ ID NO. 3 is mutated into valine and glycine at the 208^{th} site is mutated into alanine;
MT-32: serine at the 209^{th} site of the amino acid sequence of an esterase A as shown in SEQ ID NO. 3 is mutated into threonine and glutamic acid at the 177^{th} site is mutated into glutamine;
MT-33: serine at the 209^{th} site of the amino acid sequence of an esterase A as shown in SEQ ID NO. 3 is mutated into threonine and serine at the 180^{th} site is mutated into leucine;
MT-34: serine at the 209^{th} site of the amino acid sequence of an esterase A as shown in SEQ ID NO. 3 is mutated into threonine and isoleucine at the 181^{st} site is mutated into valine;
MT-35: serine at the 209^{th} site of the amino acid sequence of an esterase A as shown in SEQ ID NO. 3 is mutated into threonine and glycine at the 208^{th} site is mutated into alanine;
MT-36: serine at the 211^{th} site of the amino acid sequence of an esterase A as shown in SEQ ID NO. 3 is mutated into tyrosine and glutamic acid at the 177^{th} site is mutated into glutamine;
MT-37: serine at the 211^{th} site of the amino acid sequence of an esterase A as shown in SEQ ID NO. 3 is mutated into tyrosine and asparagine at the 178^{th} site is mutated into alanine;
MT-38: serine at the 211^{th} site of the amino acid sequence of an esterase A as shown in SEQ ID NO. 3 is mutated into tyrosine and serine at the 180^{th} site is mutated into leucine;
MT-39: serine at the 211^{th} site of the amino acid sequence of an esterase A as shown in SEQ ID NO. 3 is mutated into tyrosine and serine at the 180^{th} site is mutated into valine;
MT-40: serine at the 211^{th} site of the amino acid sequence of an esterase A as shown in SEQ ID NO. 3 is mutated into tyrosine and isoleucine at the 181^{st} site is mutated into valine;
MT-41: serine at the 211^{th} site of the amino acid sequence of an esterase A as shown in SEQ ID NO. 3 is mutated into tyrosine and glycine at the 208^{th} site is mutated into alanine; and
MT-42: serine at the 211^{th} site of the amino acid sequence of an esterase A as shown in SEQ ID NO. 3 is mutated into tyrosine and serine at the 209^{th} site is mutated into threonine.

Compared with the amino acid sequence of the esterase A as shown in SEQ ID NO. 3, the amino acid sequence of the esterase mutant involves mutation sites including at least three of the amino acid mutation sites in claim 1.

### Example 4

Experiment on polyester degradation by esterase mutant having polyester degradation activity
(1) Firstly, a polyester wafer (diameter of 6 mm) was punched out with a puncher; the polyester wafer was put into an eppendorf (EP) tube, a Triton X-100 water solution with a volume concentration of 0.5% was added, the wafer was incubated in a metal bath at 50°C for 30 min, and then the wafer was clipped out; a 10 mmol/L Na₂CO₃ water solution was added, and the wafer was incubated in the metal bath at 50°C for 30 min; the wafer was clipped out, put into ddH₂O for immersion, and incubated in the metal bath at 50°C for 30 min; and the wafer was taken out and blow-dried with nitrogen for later use.
(2) A protein MT-1 water solution (wild-type esterase (SEQ ID No. 1) as a control) with a final concentration of 500 nM and the polyester wafer obtained in step (1) were incubated in 600 µL of glycine-sodium hydroxide buffer at 50 mM and pH=9.0, a polyester hydrolysis reaction was performed at a reaction temperature of 50°C, which lasts for 18 h, the water solution was diluted with a stop buffer prepared from phosphate buffer with a volume ratio of 25% and pH of 2.5 and methanol with a volume ratio of 75% to stop the reaction, heating was performed at 85°C for 10 min, and then centrifugation was performed at 12,000 rpm for 10 min to remove inactivated proteins. A supernatant was pipetted out for high performance liquid chromatography (HPLC) analysis. All the experiments were repeated three times.

HPLC was performed on a water e2695 chromatograph, a HyPURITY C18 chromatographic column (4.6×250 mm) was prepared, where a mobile phase A was phosphate buffer solution (pH of 2.5), a mobile phase B was methanol, a flow rate was set as 0.5 mL/min, and HPLC was monitored at a wavelength of 240 nm. A program was set as follows: 0-5 min, 25% V/V B liquid; 5-25 min, 25%-100% V/V B liquid linear gradient.

MT-2, MT-3, MT-4, MT-5, MT-6, MT-7, MT-8, MT-9, MT-10, MT-11, MT-12, MT-13, MT-14, MT-15, MT-16, MT-17, MT-18, MT-19, MT-20, MT-21, MT-22, MT-23, MT-24, MT-25, MT-26, MT-27, MT-28, MT-29, MT-30, MT-31, MT-32, MT-33, MT-34, MT-35, MT-36, MT-37, MT-38, MT-39, MT-40, MT-41, and MT-42 were subjected to the experiment on polyester degradation with reference to the method in this example, and experimental results are shown in Table 10.

**Table 10 Relative degradation activity of esterase mutant having polyester degradation activity for polyesters at a pH of 9.0 and 50°C**

| Name of esterase | Relative enzymatic activity |
|---|---|
| Wild-type esterase | 1 |
| MT-1 | 59.4 |
| MT-2 | 60.9 |
| MT-3 | 67.7 |
| MT-4 | 51.5 |
| MT-5 | 60.4 |
| MT-6 | 61.4 |
| MT-7 | 34.7 |
| MT-8 | 59.9 |
| MT-9 | 65.2 |
| MT-10 | 59.6 |
| MT-11 | 68.4 |
| MT-12 | 70 |
| MT-13 | 78.6 |
| MT-14 | 82.4 |
| MT-15 | 27.9 |
| MT-16 | 44.6 |
| MT-17 | 73.9 |
| MT-18 | 72 |
| MT-19 | 70.2 |
| MT-20 | 61.4 |
| MT-21 | 73.2 |
| MT-22 | 77.9 |
| MT-23 | 72.8 |
| MT-24 | 54.4 |
| MT-25 | 72 |
| MT-26 | 52.8 |
| MT-27 | 77.7 |
| MT-28 | 52.6 |
| MT-29 | 54.4 |
| MT-30 | 40.7 |
| MT-31 | 60.6 |
| MT-32 | 61.6 |
| MT-33 | 70.4 |
| MT-34 | 66.6 |
| MT-35 | 51.4 |
| MT-36 | 72.6 |
| MT-37 | 71.5 |
| MT-38 | 76.3 |
| MT-39 | 74.5 |
| MT-40 | 71.2 |
| MT-41 | 76.5 |
| MT-42 | 73.9 |

It is shown from the experiment that a temperature of the polyester hydrolysis reaction may be selected from any value from 37°C to 70°C, such as 37°C, 40°C, 45°C, 55°C, 60°C, or 70°C, and the hydrolysis effect of the above esterase mutant for polyesters is superior to that of the wild-type esterase.

It is shown from the experiment that a pH of the glycine-sodium hydroxide buffer of the polyester hydrolysis reaction may be selected from any value from 4 to 11, such as 4, 5, 6, 7, 8, 8.5, 10, or 11, and the hydrolysis effect of the above esterase mutant for polyesters is superior to that of the wild-type esterase.

## Claims

1. Esterase mutants having polyester degradation activity, **characterized in that**, the esterase mutants are one of the following:
MT-1: glutamic acid at the 177^{th} site of the amino acid sequence of an esterase A as shown in SEQ ID NO. 3 is mutated into glutamine;
MT-2: asparagine at the 178^{th} site of the amino acid sequence of an esterase A as shown in SEQ ID NO. 3 is mutated into alanine;
MT-3: serine at the 180^{th} site of the amino acid sequence of an esterase A as shown in SEQ ID NO. 3 is mutated into threonine;
MT-4: serine at the 180^{th} site of the amino acid sequence of an esterase A as shown in SEQ ID NO. 3 is mutated into leucine;
MT-5: serine at the 180^{th} site of the amino acid sequence of an esterase A as shown in SEQ ID NO. 3 is mutated into valine;
MT-6: isoleucine at the 181^{st} site of the amino acid sequence of an esterase A as shown in SEQ ID NO. 3 is mutated into valine;
MT-7: glycine at the 208^{th} site of the amino acid sequence of an esterase A as shown in SEQ ID NO. 3 is mutated into alanine;
MT-8: serine at the 209^{th} site of the amino acid sequence of an esterase A as shown in SEQ ID NO. 3 is mutated into threonine;
MT-9: serine at the 211^{th} site of the amino acid sequence of an esterase A as shown in SEQ ID NO. 3 is mutated into tyrosine;
MT-10: glutamic acid at the 177^{th} site of the amino acid sequence of an esterase A as shown in SEQ ID NO. 3 is mutated into glutamine and serine at the 180^{th} site is mutated into leucine;
MT-11: glutamic acid at the 177^{th} site of the amino acid sequence of an esterase A as shown in SEQ ID NO. 3 is mutated into glutamine and isoleucine at the 181^{st} site is mutated into valine;
MT-12: glutamic acid at the 177^{th} site of the amino acid sequence of an esterase A as shown in SEQ ID NO. 3 is mutated into glutamine and glycine at the 208^{th} site is mutated into alanine;
MT-13: asparagine at the 178^{th} site of the amino acid sequence of an esterase A as shown in SEQ ID NO. 3 is mutated into alanine and glutamic acid at the 177th site is mutated into glutamine;
MT-14: asparagine at the 178^{th} site of the amino acid sequence of an esterase A as shown in SEQ ID NO. 3 is mutated into alanine and serine at the 180^{th} site is mutated into leucine;
MT-15: asparagine at the 178^{th} site of the amino acid sequence of an esterase A as shown in SEQ ID NO. 3 is mutated into alanine and isoleucine at the 181^{st} site is mutated into valine;
MT-16: asparagine at the 178^{th} site of the amino acid sequence of an esterase A as shown in SEQ ID NO. 3 is mutated into alanine and glycine at the 208^{th} site is mutated into alanine;
MT-17: asparagine at the 178^{th} site of the amino acid sequence of an esterase A as shown in SEQ ID NO. 3 is mutated into alanine and serine at the 209^{th} site is mutated into threonine;
MT-18: serine at the 180^{th} site of the amino acid sequence of an esterase A as shown in SEQ ID NO. 3 is mutated into threonine and glutamic acid at the 177^{th} site is mutated into glutamine;
MT-19: serine at the 180^{th} site of the amino acid sequence of an esterase A as shown in SEQ ID NO. 3 is mutated into threonine and asparagine at the 178^{th} site is mutated into alanine;
MT-20: serine at the 180^{th} site of the amino acid sequence of an esterase A as shown in SEQ ID NO. 3 is mutated into threonine and isoleucine at the 181^{st} site is mutated into valine;
MT-21: serine at the 180^{th} site of the amino acid sequence of an esterase A as shown in SEQ ID NO. 3 is mutated into threonine and glycine at the 208^{th} site is mutated into alanine;
MT-22: serine at the 180^{th} site of the amino acid sequence of an esterase A as shown in SEQ ID NO. 3 is mutated into threonine and serine at the 209^{th} site is mutated into threonine;
MT-23: serine at the 180^{th} site of the amino acid sequence of an esterase A as shown in SEQ ID NO. 3 is mutated into threonine and serine at the 211^{th} site is mutated into tyrosine;
MT-24: serine at the 180^{th} site of the amino acid sequence of an esterase A as shown in SEQ ID NO. 3 is mutated into leucine and isoleucine at the 181^{st} site is mutated into valine;
MT-25: serine at the 180^{th} site of the amino acid sequence of an esterase A as shown in SEQ ID NO. 3 is mutated into leucine and glycine at the 208^{th} site is mutated into alanine;
MT-26: serine at the 180^{th} site of the amino acid sequence of an esterase A as shown in SEQ ID NO. 3 is mutated into valine and glutamic acid at the 177^{th} site is mutated into glutamine;
MT-27: serine at the 180^{th} site of the amino acid sequence of an esterase A as shown in SEQ ID NO. 3 is mutated into valine and asparagine at the 178^{th} site is mutated into alanine;
MT-28: serine at the 180^{th} site of the amino acid sequence of an esterase A as shown in SEQ ID NO. 3 is mutated into valine and isoleucine at the 181^{st} site is mutated into valine;
MT-29: serine at the 180^{th} site of the amino acid sequence of an esterase A as shown in SEQ ID NO. 3 is mutated into valine and glycine at the 208^{th} site is mutated into alanine;
MT-30: serine at the 180^{th} site of the amino acid sequence of an esterase A as shown in SEQ ID NO. 3 is mutated into valine and serine at the 209^{th} site is mutated into threonine;
MT-31: isoleucine at the 181^{st} site of the amino acid sequence of an esterase A as shown in SEQ ID NO. 3 is mutated into valine and glycine at the 208^{th} site is mutated into alanine;
MT-32: serine at the 209^{th} site of the amino acid sequence of an esterase A as shown in SEQ ID NO. 3 is mutated into threonine and glutamic acid at the 177^{th} site is mutated into glutamine;
MT-33: serine at the 209^{th} site of the amino acid sequence of an esterase A as shown in SEQ ID NO. 3 is mutated into threonine and serine at the 180^{th} site is mutated into leucine;
MT-34: serine at the 209^{th} site of the amino acid sequence of an esterase A as shown in SEQ ID NO. 3 is mutated into threonine and isoleucine at the 181^{st} site is mutated into valine;
MT-35: serine at the 209^{th} site of the amino acid sequence of an esterase A as shown in SEQ ID NO. 3 is mutated into threonine and glycine at the 208^{th} site is mutated into alanine;
MT-36: serine at the 211^{th} site of the amino acid sequence of an esterase A as shown in SEQ ID NO. 3 is mutated into tyrosine and glutamic acid at the 177^{th} site is mutated into glutamine;
MT-37: serine at the 211^{th} site of the amino acid sequence of an esterase A as shown in SEQ ID NO. 3 is mutated into tyrosine and asparagine at the 178^{th} site is mutated into alanine;
MT-38: serine at the 211^{th} site of the amino acid sequence of an esterase A as shown in SEQ ID NO. 3 is mutated into tyrosine and serine at the 180^{th} site is mutated into leucine;
MT-39: serine at the 211^{th} site of the amino acid sequence of an esterase A as shown in SEQ ID NO. 3 is mutated into tyrosine and serine at the 180^{th} site is mutated into valine;
MT-40: serine at the 211^{th} site of the amino acid sequence of an esterase A as shown in SEQ ID NO. 3 is mutated into tyrosine and isoleucine at the 181^{st} site is mutated into valine;
MT-41: serine at the 211^{th} site of the amino acid sequence of an esterase A as shown in SEQ ID NO. 3 is mutated into tyrosine and glycine at the 208^{th} site is mutated into alanine; and
MT-42: serine at the 211^{th} site of the amino acid sequence of an esterase A as shown in SEQ ID NO. 3 is mutated into tyrosine and serine at the 209^{th} site is mutated into threonine.

2. The esterase mutant having polyester degradation activity according to claim 1, **characterized in that**, compared with the amino acid sequence of the esterase A as shown in SEQ ID NO. 3, involves the mutation sites involved in the amino acid sequence of the esterase mutant include at least three of the amino acid mutation sites.

3. Recombinant plasmids of encoding genes for the esterase mutants having polyester degradation activity according claim 1 or 2.

4. Recombinant strains containing the recombinant plasmids according to claim 3.

5. Use of the esterase mutants having polyester degradation activity according claim 1 or 2 in polyester hydrolysis.
